# EUROPEAN PATENT SPECIFICATION

(11) **EP 1 194 371 B1**
(45) Date of publication and mention of the grant of the patent: **18.03.2009**
(21) Application number: 99966077.2
(22) Date of filing: 09.12.1999
(51) Int. Cl.: C01B 7/09, C02F 1/76, A61L 2/18, C11D 3/395

(54) **METHOD OF MANUFACTURE OF STABLE OXIDIZING BROMINE FORMULATIONS**
VERFAHREN ZUR HERSTELLUNG VON STABILEN OXIDIERENDEN BROMFORMULIERUNGEN
PROCEDE DE FABRICATION DE FORMULATIONS STABILISEES D'OXYDANTS BROMES

(30) Priority: 21.04.1999 US 296212
(43) Date of publication of application: 10.04.2002
(73) Proprietor: NALCO CHEMICAL COMPANY, Naperville Illinois 60563-1198 (US)
(72) Inventor: YANG, Shunong, Naperville, IL 60565 (US); MCCOY, William, F., Naperville, IL 60540 (US); DALLMIER, Anthony, W., Aurora, IL 60504 (US)
(74) Representative: Harrison Goddard Foote
(86) International application number: PCT/US1999/029192
(87) International publication number: WO 2000/064806

(56) References cited:
- WO-A-99/55627
- US-A- 3 558 503
- US-A- 3 558 503
- US-A- 4 131 556
- US-A- 5 415 803
- US-A- 5 795 487
- US-A- 5 817 888
- US-A- 5 942 126
- US-A- 6 007 726
- US-A- 6 156 229

## Description

### FIELD OF THE INVENTION

The present invention relates to formulations used in biofouling control in industrial water systems. More specifically, the present invention relates to methods of preparing stable oxidizing bromine formulations and their use in biofouling control in industrial water systems.

### BACKGROUND OF THE INVENTION

While elemental liquid bromine is an effective biocide, its low solubility (<4 g/ 100 g water), low boiling point (54.3°C), high vapor pressure (214 mm Hg at 25°C) and extreme corrosivity limit its use as a biocide in industrial applications. Another oxidizing bromine compound, bromate, has very little antimicrobial activity. Bromate is also very toxic to mammals and is a suspected carcinogen. Nonoxidizing inorganic bromine compounds, such as bromide, have little or no antimicrobial activity.

A mixture of an aqueous bromine solution and a bromine stabilizer has been used to generate stable oxidizing bromine compounds for use as a biocide. An unstabilized aqueous bromine solution is very acidic, unstable and emits very pungent bromine fumes. The concentration of stabilized hypobromite solution that can be made from liquid bromine, however, has been limited due to the low solubility of bromine in water.

US 3,558,503 discloses a process for the production of stable bromine solutions which comprises treating aqueous solutions of bromine with a nitrogen-containing bromine stabilizer and an alkali metal or alkaline earth hydroxide at controlled pH levels. Suitable bromine stabilizers comprise biuret, succinimide, urea and lower aliphatic mono- and disubstituted ureas, sulfamic acid and alkyl sulfonamides. The order of addition of the stabilized bromine stabilizer solution and the metal hydroxide to water or an aqueous solution of other reagents is not critical.

It has also been suggested that, in addition to a bromine stabilizer, an oxidizer, such as hypochlorite, be added to activate the bromide to hypobromite. After the completion of the conversion of bromide to hypobromite, the hypobromite is stabilized by the addition of a halogen stabilizer, such as sulfamate. While this is an improved process with a higher level of oxidizing halogen content (around 14% as Br₂), this process still requires the separate step of synthesizing sodium hypobromite (NaOBr) as a bromine source. NaOBr is known to be very unstable and will rapidly disproportionate to bromide and bromate, both of which have little or no antimicrobial activity. In addition, because sodium hypochlorifie (NaOCl) is used as an activation agent, the concentration of stabilized product is limited by the available concentration of NaOCl.

Also known are methods of generating bromine for onsite use. Such processes involve electrolytically converting bromate into active bromine compounds such as bromine, hypobromous acid, hypobromite ion and hydrogen tribromide under acidic conditions. However, because the above process generates bromine for on-site use, methods or measures for optimizing bromine stabilization are not addressed.

Therefore, methods of generating higher concentrations of stable oxidizing bromine formulations in a safer manner are needed.

### SUMMARY OF THE INVENTION

The present invention satisfies the aforementioned needs by providing a method of generating a stable oxidizing bromine compound which includes the steps of:
preparing a caustic solution comprising a halogen stabilizer, water and an alkali or alkaline earth metal hydroxide, the halogen stabilizer being selected from the group consisting of R-NH₂, R-NH-R¹, R-SO₂-NH₂, R-SO₂-NHR¹, R-CO-NH₂,R-CO-NH-R¹ and R-CO-NH-CO-R¹ wherein R is a hydroxy group, an alkyl group or an aromatic group and R¹ is an alkyl group or an aromatic group,
adding bromine chloride to the solution while agitating the solution, and
cooling the solution.

Preferred halogen stabilizers include saccharin, benzenesulfonamide urea, thiourea, creatinine, cyanuric acids, alkyl hydantoins, mono or di ethanolamine, organic sulfonamides, biuret, sulfamic acid, organic sulfamates and melamine.

In an embodiment, the caustic solution has a pH greater than 13 after the addition of the bromine chloride.

In an embodiment, the step of adding bromine chloride is further characterized as adding bromine chloride in a molar amount approximately equal to the molar amount of halogen stabilizer and approximately equal to one-half of the molar amount of alkali or alkaline earth metal hydroxide.

In an embodiment, the solution is cooled to a temperature of less than 25°C.

In an embodiment, the step of adding bromine chloride is performed without exposing the bromine chloride to air.

In an embodiment, an alkali or alkaline earth metal hydroxide is added to the solution after the addition of bromine chloride to increase the pH of the solution above 13.

In an embodiment, the present invention provides a method of generating a stable oxidizing bromine compound, the method comprising the following steps:
preparing a caustic solution comprising a halogen stabilizer, water and an alkali or alkaline earth metal hydroxide, the halogen stabilizer being selected from the group consisting of saccharin, benzenesulfonamide, urea, thiourea, creatinine, cyanuric acids, alkyl hydantoins, monoethanolamine, diethanolamine, organic sulfonamides, biuret, sulfamic acid, organic sulfamates and melamine,
adding bromine chloride to the solution in a molar amount approximately equal to a molar amount of halogen stabilizer and approximately one-half of a molar amount of alkali or alkaline earth metal hydroxide and without exposing the bromine chloride to air,
mixing the solution,
cooling the solution to a temperature of less than 25°C, and
adding an alkali or alkaline earth metal hydroxide to the solution to increase the pH of the solution above 13.

The method of the present invention thus facilitates the preparation of an aqueous biocide solution containing a stable oxidizing bromine formulation. The solution comprises at least one oxidizing bromine compound selected from the group consisting of SO₃NHBr and SO₃NBr₂ when sulfamate is used as the bromine stabilizer and a base in an amount sufficient to raise the pH of the solution to a level greater than 13, wherein the base in the solution is an alkali or alkaline earth metal hydroxide.

It is therefore an advantage of the present invention to generate a stable oxidizing bromine containing solution using bromine chloride in a safe and efficient manner whereby no bromine fumes are generated.

It is another advantage of the present invention to generate a higher concentration of stabilized hypobromite without the need for a separate step for hypobromite generation.

Another advantage of the present invention is that it provides a method for generating water soluble solid stable oxidizing bromine compounds.

Still another advantage of the present invention is that it provides a method for generating stable oxidizing bromine compounds without unwanted by-products such as high levels of bromate.

Still another advantage of the present invention is that the method of the present invention does not generate chloride and therefore the method of the present invention provides stable oxidizing bromine formulations that are less corrosive.

Yet another advantage of the present invention is that it provides stable oxidizing bromine compounds that are safer to transport and that are non-acidic.

Yet another advantage of the present invention is that it generates stable oxidizing bromine compounds for biofouling control in industrial water systems that are more compatible with other water treatment chemicals than unstabilized oxidizing bromine compounds.

The industrial water systems include cooling water systems, cooling ponds, reservoirs, sweetwater applications, decorative fountains, pasteurizers, evaporative condensers, hydrostatic sterilizers and retorts, gas scrubber systems and air washer systems.

Another advantage of the present invention is that it provides an improved method of biofouling control in pulp and paper processing systems.

Another advantage of the present invention is that it provides an improved method of biofouling control occurring on the surfaces of equipment in contact with produced oil field waters.

Another advantage of the present invention is that it provides an improved method of biofouling control in a food processing system.

Yet another advantage of the present invention is that it provides improved biofouling control in a beverage processing system.

Still another advantage of the present invention is that it provides improved biofouling control in a recreational water system.

Another advantage of the present invention is that it provides an improved method of disinfecting a hard surface.

Another advantage of the present invention is that it provides an improved bleaching method for the laundering of soiled garments and for the manufacture of cellulosic materials.

And, another advantage of the present invention is that it provides an improved method of washing food items, such as fruit and other food items.

Other objects and advantages of the present invention will be apparent upon a review of the following detailed description and appended claims.

### DETAILED DESCRIPTION OF THE INVENTION

The present invention provides a plurality of formulations and methods for generating a wide concentration of stable oxidizing bromine compounds for biofouling control in cooling water and other industrial systems.

The strategy employed by the present invention utilizes bromine chloride as the bromine source, and this material also serves as an oxidizing agent. The solution is then cooled to a temperature preferably of less than 25°C and even more preferably of less than 10°C. An acidic stabilizer or acidic stabilizing solution, such as sulfamic acid, is then added to the solution to lower the pH of the solution to less than 2. Additional stabilizer is then added to achieve equal molar amounts relative to bromine for optimal stabilization. Without being limited by theory, the following reactions are believed to occur:

H-O-SO₂-NH₂ ---> H⁺ + ⁻O-SO₂-NH₂ (1)

2 Br⁻ + BrO₃⁻ + 3 H⁺ → 3HBrO (2)

HBrO + -O-SO₂-NH₂ ---> ⁻O-SO₂-NH-Br, ⁻O-SO₂-NBr₂ and other stable oxidizing bromine compounds (3)

Since bromide, bromate and sulfamate co-exist in the resulting solution, reaction (1) to reaction (3) occurs sequentially with respect to each other. Without being limited by theory, the existence of an oxidizing bromine stabilizer and correct bromide to bromate ratio are believed to prevent the formation of bromine according to the following reaction:

5 NaBr + NaBrO₃ + 6H⁺ ---> 3 Br₂ + 6 Na⁺ + 3 H₂O (4)

If reaction (4) were to happen instead of reaction (2), half of the raw bromine source would convert back to non-biocidal and non-oxidizing bromide according to reaction (5):

Br₂ + H₂O → HBrO + HBr (5)

However, an analysis of products prepared in accordance with the present invention confirms that the reaction yield is higher than 50%. In fact, more than 80% of the bromine source was converted to oxidized bromine forms. Accordingly, the reaction yield of at least 80% was achieved.

The reaction time for reactions 1-3 at a pH of less than 2 ranges from 5 to 10 minutes with good agitation. If the product is not going to be used immediately, a strong base, such as NaOH, is added to raise the product pH to a level greater than 13 making the product thermally stable. During the pH adjustment, temperature control is important because the temperature increase by the heat generated from the acid-base reaction can cause the product to decompose. Accordingly, cooling may be necessary.

The product made with the above process has good thermostability and a high total available halogen concentration, as high as 34% as Br₂.

The stable oxidizing bromine compound of the present invention can be used to provide improved biofouling control in industrial water systems, pulp and paper processing systems, food and beverage processing systems and recreational water systems. The stable oxidizing bromine compound of the present invention can also be used as a bleaching agent and to disinfect a hard surface. By way of example only, the present invention may be added to an aqueous media used to transport food through various processing systems and also to disinfect process equipment and waste water streams.

It should be understood that various changes and modifications to the presently preferred embodiments described herein will be apparent to those skilled in the art. Such changes and modifications can be made without departing from the scope of the present invention and without diminishing its attendant advantages. It is therefore intended that such changes and modifications be covered by the appended claims.

## Claims

1. A method of generating a stable oxidizing bromine compound, the method comprising the following steps:
preparing a caustic solution comprising a halogen stabilizer, water and an alkali or alkaline earth metal hydroxide, the halogen stabilizer being selected from the group consisting of R-NH₂, R-NH-R¹, R-SO₂-NH2, R-SO₂-NHR¹, R-CO-NH₂, R-CO-NH-R¹ and R-CO-NH-CO-R¹ wherein R is a hydroxy group, an alkyl group or an aromatic group and R¹ is an alkyl group or an aromatic group,
adding bromine chloride to the solution while mixing the solution, and
cooling the solution.

2. The method of claim 1 wherein the halogen stabilizer is selected from the group consisting of saccharin, benzenesulfonamide, urea, thiourea, creatinine, cyanuric acids, alkyl hydantoins, monoethanolamine, diethanolamine, organic sulfonamides, biuret, sulfamic acid, organic sulfamates and melamine.

3. The method of claim 1 or 2 wherein the caustic solution has a pH after the addition of bromine chloride of greater than 13.

4. The method of claim 1, 2 or 3 wherein the step of adding bromine chloride is further **characterized** as adding said compound in a molar amount approximately equal to a molar amount of halogen stabilizer and approximately one-half of a molar amount of alkali or alkaline earth metal hydroxide.

5. The method of any one of claims 1 to 4 wherein the cooling step is further **characterized** as cooling the solution to a temperature of less than 25°C.

6. The method of any preceding claim wherein the step of adding bromine chloride is performed without exposing the bromine chloride to air.

7. The method of claim 1 or 2 further comprising the following step after the addition of the bromine chloride:
adding an alkali or alkaline earth metal hydroxide to the solution to increase the pH of the solution above 13.

8. A method of generating a stable oxidizing bromine compound, the method comprising the following steps:
preparing a caustic solution comprising a halogen stabilizer, water and an alkali or alkaline earth metal hydroxide, the halogen stabilizer being selected from the group consisting of saccharin, benzenesulfonamide, urea, thiourea, creatinine, cyanuric acids, alkyl hydantoins, monoethanolamine, diethanolamine, organic sulfonamides, biuret, sulfamic acid, organic sulfamates and melamine,
adding bromine chloride to the solution in a molar amount approximately equal to a molar amount of halogen stabilizer and approximately one-half of a molar amount of alkali or alkaline earth metal hydroxide and without exposing the bromine chloride to air,
mixing the solution,
cooling the solution to a temperature of less than 25°C, and
adding an alkali or alkaline earth metal hydroxide to the solution to increase the pH of the solution above 13.

## Patentansprüche

1. Verfahren zum Erzeugen einer stabilen oxidierenden Bromverbindung, wobei das Verfahren die folgenden Schritte umfasst:
das Herstellen einer Ätzlösung, umfassend einen Halogenstabilisator, Wasser und ein Alkali- oder Erdalkalimetallhydroxid, wobei der Halogenstabilisator ausgewählt ist aus der Gruppe bestehend aus R-NH₂, R-NH-R¹, R-SO₂-NH₂, R-SO₂-NHR¹, R-CO-NH₂, R-CO-NH-R¹ und R-CC-NH-CO-R¹, worin R eine Hydroxygruppe, eine Alkylgruppe oder eine aromatische Gruppe ist und R¹ eine Alkylgruppe oder eine aromatische Gruppe ist,
das Zugeben von Bromchlorid zu der Lösung, während die Lösung gemischt wird, und
das Kühlen der Lösung.

2. Verfahren nach Anspruch 1, wobei der Halogenstabilisator ausgewählt ist aus der Gruppe bestehend aus Saccharin, Benzolsulfonamid, Harnstoff, Thioharnstoff, Kreatinin, Cyanursäuren, Alkylhydantoinen, Monoethanolamin, Diethanolamin, organischen Sulfonamiden, Biuret, Sulfaminsäure, organischen Sulfamaten und Melamin.

3. Verfahren nach Anspruch 1 oder 2, wobei die Ätzlösung nach der Zugabe von Bromchlorid einen pH von mehr als 13 aufweist.

4. Verfahren nach Anspruch 1, 2 oder 3, wobei der Schritt des Zugebens von Bromchlorid ferner **dadurch gekennzeichnet ist, dass** die Verbindung in einer molaren Menge zugegeben wird, die ungefähr einer molaren Menge an Halogenstabilisator und ungefähr der Hälfte einer molaren Menge an Alkali- oder Erdalkalimetallhydroxid entspricht.

5. Verfahren nach einem der Ansprüche 1 bis 4, wobei der Kühlungsschritt ferner **dadurch gekennzeichnet ist, dass** die Lösung auf eine Temperatur von weniger als 25 °C gekühlt wird.

6. Verfahren nach einem vorangehenden Anspruch, wobei der Schritt des Zugebens von Bromchlorid durchgeführt wird, ohne das Bromchlorid der Luft auszusetzen.

7. Verfahren nach Anspruch 1 oder 2, außerdem umfassend den folgenden Schritt nach der Zugabe des Bromchlorids. das Zugeben eines Alkali- oder Erdalkalimetallhydroxids zu der Lösung, um den pH der Lösung auf mehr als 13 zu erhöhen.

8. Verfahren zum Erzeugen einer stabilen oxidierenden Bromverbindung, wobei das Verfahren die folgenden Schritte umfasst:
das Herstellen einer Ätzlösung, umfassend einen Halogenstabilisator, Wasser und ein Alkali- oder Erdalkalimetallhydroxid, wobei der Halogenstabilisator ausgewählt ist aus der Gruppe bestehend aus Saccharin, Benzolsulfonamid, Harnstoff, Thioharnstoff, Kreatinin, Cyanursäuren, Alkylhydantoinen, Monoethanolamin, Diethanolamin, organischen Sulfonamiden, Biuret, Sulfaminsäure, organischen Sulfamaten und Melamin,
das Zugeben von Bromchlorid zu der Lösung in einer molaren Menge, die ungefähr einer molaren Menge an Halogenstabilisator und ungefähr der Hälfte einer molaren Menge an Alkali- oder Erdalkalimetallhydroxid entspricht, und ohne das Bromchlorid der Luft auszusetzen,
das Mischen der Lösung,
das Kühlen der Lösung auf eine Temperatur von weniger als 25 °C, und
das Zugeben eines Alkali- oder Erdalkalimetallhydroxids zu der Lösung, um den pH der Lösung auf mehr als 13 zu erhöhen.

## Revendications

1. Procédé de formation d'un composé oxydant bromé stabilisé, le composé comprenant les étapes suivantes :
préparation d'une solution caustique comprenant un stabilisant d'halogènes, de l'eau et un hydroxyde de métal alcalin ou alcalino-terreux, le stabilisant d'halogènes étant choisi dans le groupe constitué de R-NH₂, R-NH-R¹, R-SO₂-NH₂, R-SO₂-NHR¹, R-CO-NH₂, R-CO-NH-R¹ et R-CO-NH-CO-R¹, où R est un groupement hydroxy, un groupement alkyle ou un groupement aromatique, et R¹ est un groupement alkyle ou un groupement aromatique,
ajout de chlorure de brome à la solution tout en mélangeant la solution, et
refroidissement de la solution.

2. Procédé selon la revendication 1, dans lequel le stabilisant d'halogènes est choisi dans le groupe constitué de la saccharine, le benzènesulfonamide, l'urée, la thiourée, la créatinine, les acides cyanuriques, les alkyl-hydantoïnes, la monoéthanolamine, la diéthanolamine, les sulfonamides organiques, le biuret, l'acide sulfamique, les sulfamates organiques et la mélamine.

3. Procédé selon la revendication 1 ou 2, dans lequel la solution caustique a un pH supérieur à 13 après l'ajout du chlorure de brome.

4. Procédé selon la revendication 1, 2 ou 3, dans lequel l'étape d'ajout du chlorure de brome est ***caractérisée*** de plus comme l'ajout dudit composé selon une quantité molaire approximativement égale à une quantité molaire du stabilisant d'halogènes et approximativement à la moitié d'une quantité molaire d'hydroxyde de métal alcalin ou alcalino-terreux.

5. Procédé selon l'une quelconque des revendications 1 à 4, dans lequel l'étape de refroidissement est ***caractérisée*** de plus comme le refroidissement de la solution à une température inférieure à 25°C.

6. Procédé selon l'une quelconque des revendications précédentes, dans lequel l'étape d'ajout du chlorure de brome est effectuée sans exposer le chlorure de brome à l'air.

7. Procédé selon la revendication 1 ou 2, comprenant de plus l'étape suivante après l'ajout du chlorure de brome :
ajout d'un hydroxyde de métal alcalin ou alcalino-terreux à la solution pour augmenter le pH de la solution au-dessus de 13.

8. Procédé de formation d'un composé oxydant bromé stabilisé, le composé comprenant les étapes suivantes :
préparation d'une solution caustique comprenant un stabilisant d'halogènes, de l'eau et un hydroxyde de métal alcalin ou alcalino-terreux, le stabilisant d'halogènes étant choisi dans le groupe constitué de la saccharine, le benzènesulfonamide, l'urée, la thiourée, la créatinine, les acides cyanuriques, les alkyl-hydantoïnes, la monoéthanolamine, la diéthanolamine, les sulfonamides organiques, le biuret, l'acide sulfamique, les sulfamates organiques et la mélamine,
l'ajout de chlorure de brome à la solution selon une quantité molaire approximativement égale à une quantité molaire du stabilisant d'halogènes et approximativement à la moitié d'une quantité molaire d'hydroxyde de métal alcalin ou alcalino-terreux et sans exposition du chlorure de brome à l'air,
le mélange de la solution,
le refroidissement de la solution à une température inférieure à 25°C, et
l'ajout d'un hydroxyde de métal alcalin ou alcalino-terreux à la solution pour augmenter le pH de la solution au-dessus de 13.
